# FASCICULE DE BREVET EUROPEEN

(11) **EP 1 814 524 B1**
(45) Date de publication et mention de la délivrance du brevet: **18.04.2012**
(21) Numéro de dépôt: 05819412.7
(22) Date de dépôt: 21.11.2005
(51) Int. Cl.: A61K 9/16, A61K 9/20, A61K 47/44, A61K 47/32, A61K 47/36

(54) **FORME PHARMACEUTIQUE ORALE MICROPARTICULAIRE SOLIDE CONCUE POUR EVITER LE MESUSAGE**
FESTE ORALE MIKROPARTIKELFÖRMIGE DARREICHUNGSFORM ZUM SCHUTZ VOR MISSBRAUCH
SOLID, ORAL, MICROPARTICULATE DOSAGE FORM WHICH HAS BEEN DESIGNED TO PREVENT MISUSE

(30) Priorité: 24.11.2004 FR 0452744
(43) Date de publication de la demande: 08.08.2007
(73) Titulaire: FLAMEL TECHNOLOGIES, 69200 Vénissieux (FR)
(72) Inventeur: SOULA, Gérard, F-69330 MEYZIEU (FR); GUIMBERTEAU, Florence, F-33450 MONTUSSAN (FR)
(74) Mandataire: Fleurance, Raphaël
(86) Numéro de dépôt international: PCT/FR2005/050973
(87) Numéro de publication internationale: WO 2006/056713

(56) Documents cités:
- EP-A- 0 198 769
- WO-A-2005/016313
- WO-A-2005/016314
- US-A- 4 070 494
- US-A1- 2002 068 085
- US-B1- 6 309 668

## Description

### Domaine de l'invention

Le domaine de la présente invention est celui des formes pharmaceutiques orales microparticulaires solides dont la composition permet d'éviter le mésusage du principe actif pharmaceutique (PA) qu'elles contiennent.

Les principes actifs considérés (PA) sont des PA pharmaceutiques, par exemple ceux classés dans la catégorie des produits stupéfiants. Ces derniers sont ceux dont l'abus peut donner lieu à des conduites toxicomaniaques.

Au sens du présent exposé, l'expression "PA", désigne aussi bien un seul principe actif, qu'un mélange de plusieurs principes actifs.

Par forme pharmaceutique microparticulaire, on entend au sens de la présente invention toute forme dans laquelle le PA est contenu dans des microparticules de taille inférieure à 1000 microns. Ces particules contenant le PA peuvent être des microcapsules à libération modifiée de PA. Dans ce dernier cas, les microcapsules sont, par exemple, enrobées d'un film polymère qui contrôle la vitesse de libération du PA après administration par voie orale.

Le but visé par la présente invention est de prévenir le détournement des médicaments solides oraux, pour tout autre usage que l'usage ou les usages thérapeutiques officiellement approuvés par les autorités de santé publique compétentes. En d'autres termes, il s'agit d'éviter le mésusage volontaire ou involontaire des médicaments solides oraux.

### Position du Problème

Le mésusage se rencontre principalement dans les cas suivants:
a. comportement addictif (toxicomanie, dopage),
b. comportement criminel (asservissement chimique).
c. utilisation d'un médicament de façon non conforme aux recommandations médicales (posologie), par mégarde ou du fait d'invalidités affectant le patient,
d. automédication.

Dans le cas a. (voire b.), les personnes ayant l'intention de faire un mésusage du médicament solide, oral, vont généralement s'employer à le mettre soit sous une forme pulvérulente pouvant être inhalée, soit sous une forme liquide injectable à l'aide d'une seringue.

L'obtention d'une forme liquide injectable à partir d'un médicament oral solide, passe par une étape d'extraction aqueuse ou alcoolique du PA visé. Cette extraction est généralement précédée d'un broyage.

Les modes d'administration par inhalation ou par injection, conviennent particulièrement bien aux toxicomanes car ce sont des modes qui permettent d'accentuer les effets du PA et qui favorisent son absorption dans l'organisme sur des temps courts. Lorsque cette poudre est aspirée par le nez ou dissoute dans de l'eau et injectée, les effets recherchés, dopants ou euphorisants, du PA, se manifestent très rapidement et de manière exacerbée.

Le mésusage de médicaments oraux solides peut également être observé lorsque le médicament est mastiqué avant d'être avalé au lieu d'être avalé rapidement conformément à la posologie. Les risques liés aux comportements addictifs (a.) et criminels (b.) et à l'automédication (d.)/ sont évidents. On rappellera que le mésusage de médicaments par injection est aggravant: les excipients peuvent être responsables de nécroses locales des tissus, d'infections, de troubles respiratoires et cardiaques.

S'agissant des déviations (c.) de l'usage d'un médicament liées à l'inattention et/ou à des invalidités du patient, elles peuvent aussi avoir des conséquences sérieuses. Par exemple, la mastication avant déglutition de formes à libération modifiée de PA, transforme le médicament en une forme à libération immédiate. Ainsi, au mieux le médicament est inefficace après un temps très court et au pire il devient toxique.

Il existe donc clairement un grave problème de santé publique lié au mésusage des médicaments, et en particulier des médicaments oraux solides.

Ce phénomène en croissance inquiète de plus en plus les autorités sanitaires qui multiplient les appels au développement de formes médicamenteuses permettant la prévention du détournement.

### Art antérieur

A la connaissance de la demanderesse, les seules tentatives de réponse à ce problème ont consisté à adjoindre aux médicaments concernés des composés chimiquement actifs contre le mésusage.

Cette solution présente des dangers certains pour les utilisateurs, y compris pour un emploi dans les conditions approuvées. De surcroît, les combinaisons de PA et d'autres composés actifs sont délicats à maîtriser et posent un problème grave de santé publique.

L'US-A-2003/0068371 décrit une formulation pharmaceutique orale comprenant un PA opiacé, un antagoniste de ce PA et un agent gélifiant (e.g. gomme xanthane). L'agent gélifiant est présenté, comme conférant à la formulation une viscosité telle qu'elle ne puisse pas être administrable par voie nasale et parentérale. La présence de l'antagoniste est un inconvénient majeur, au regard des risques médicaux éventuellement encourus par les utilisateurs. En outre, cette forme pharmaceutique peut être mise sous forme pulvérulente et, par conséquent peut être l'objet d'un mésusage par voie nasale.

EP-A-0 198 769 décrit une forme pharmaceutique dont la composition permet la délivrance de son contenu sur une longue période. Grâce à un système flottant permettant d'accroitre le temps de résidence gastrique de la forme pharmaceutique, le temps d'absorption du principe actif (PA) contenu dans ladite forme pharmaceutique est étendu dans le temps. Les tablettes flottantes comprennent un principe actif thérapeutique, un agent gélifiant ainsi qu'une huile inerte acceptable au plan thérapeutique et de l'eau. Cependant, une telle forme pharmaceutique ne permet pas d'éviter un mésusage.

WO 2005/016314 décrit des formes pharmaceutiques anti-abus thermoformées sans extrusion contenant, en plus d'un ou plusieurs principes actifs potentiellement capables d'usage abusif, ainsi qu'optionnellement des adjuvants physiologiquement acceptables, au moins un polymère naturel ou synthétique et optionnellement au moins une cire, le polymère naturel ou synthétique et la cire optionnelle ayant une résistance à la rupture d'au moins 500 N.

WO 2005/016313 décrit des formes pharmaceutiques anti-abus thermoformées sans changement de couleur par extrusion, contenant, en plus d'un ou plusieurs principes actifs potentiellement capables d'usage abusif, ainsi qu'optionnellement des adjuvants physiologiquement acceptables, au moins un polymère naturel ou synthétique et optionnellement au moins une cire, le polymère naturel ou synthétique et la cire optionnelle ayant une résistance à la rupture d'au moins 500 N.

### Objectifs de l'invention

Dans ces circonstances, l'un des objectifs essentiels de la présente invention est de combler les lacunes de l'art antérieur.

Un autre objectif essentiel de l'invention est de fournir de nouveaux médicaments solides oraux, dont le mésusage sera rendu très difficile voire impossible, notamment pour les cas (a.)(b.)(c.)(d.) susévoqués, et ce sans recourir à des substances, autres que le PA, pouvant être pharmaceutiquement actives et donc dangereuses pour les utilisateurs.

Un autre objectif essentiel de l'invention est de fournir un nouveau médicament solide oral, permettant d'éviter le détournement frauduleux des propriétés du PA qu'il contient, en empêchant toute transformation du médicament donnant accès à des prises par les voies orales, nasales et/ou injectables (intra-veineuse, sous-cutanée, intra-musculaire, ...) hors du cadre thérapeutique. Ce faisant les risques associés à ces dérives seraient prévenus ou à tout le moins fortement réduits.

Un autre objectif-essentiel de l'invention est de fournir un nouveau médicament solide oral, permettant d'éviter le mésusage tout en garantissant pour le patient normalement suivi une qualité de traitement, en particulier une dose, conforme à ses besoins.

Un autre objectif essentiel de l'invention est de fournir un nouveau médicament solide oral, permettant d'éviter le mésusage, sans affecter les propriétés pharmacologiques du médicament, et sans faire courir de risques supplémentaires au patient utilisant normalement le médicament et enfin sans nuire au confort de ce dernier lors de l'administration.

Un autre objectif essentiel de l'invention est de fournir un nouveau médicament solide oral, permettant d'éviter le mésusage, qui soit simple à obtenir et qui ne grève pas son coût de revient.

### Description succincte de l'invention

Pour atteindre ces objectifs, les inventeurs ont eu le mérite de reformuler le problème général du mésusage des formes pharmaceutiques microparticulaires.

Si on examine les différents modes d'administration illicites d'un principe actif, il apparaît en effet que le broyage de la forme sèche microparticulaire est une étape obligée.

En effet, dans le cas d'un mésusage par administration parentérale, il est nécessaire de procéder au préalable à l'extraction du PA dans une phase liquide, en pratique de l'eau, et ceci à une concentration suffisamment élevée pour éviter d'injecter des volumes trop élevés, par exemple supérieurs à 1 ml. Cette étape d'extraction nécessite au préalable de broyer la forme sèche microparticulaire afin de permettre la dissolution ou la mise en suspension du princpe actif.

Dans le cas d'un mésusage par administration nasale, la forme pharmaceutique microparticulaire sèche doit au préalable être transformée sous forme d'une poudre pulvérulente apte à l'aspiration. De nouveau le broyage de la forme microparticulaire est une étape obligée.

Dans le cas d'un mésusage par administration orale de la forme microparticulaire sèche, il est nécessaire d'accélérer la libération du principe actif en broyant finement les microparticules.

Ainsi, le broyage d'une forme microparticulaire sèche est une étape obligée pour le mésusage de ladite forme pharmaceutique.

Il est du mérite de la Demanderesse d'avoir reformulé le problème de la lutte contre le mésusage des formes pharmaceutiques microparticulaires sèches en un problème d'empêchement du broyage des microparticules contenant le PA.

Cette nouvelle approche lui a permis de découvrir, de manière surprenante et inattendue, qu'il convient de faire intervenir, dans la composition du médicament dont on cherche à empêcher le mésusage, une combinaison d'excipients pharmaceutiquement acceptables dont le mode d'action physico-chimique permet de contrarier, voire de rendre impossible, tout acte volontaire ou non de mésusage.

C'est ainsi que l'invention concerne, à titre principal, une forme médicamenteuse orale et solide, caractérisée en ce qu'au moins une partie du PA qu'elle comprend est contenu dans des microparticules et en ce qu'elle comprend des moyens anti-broyage prévus pour rendre difficile voire impossible le broyage des microparticules de PA, de manière à éviter le mésusage.

Les moyens anti-broyage de cette forme pharmaceutique microparticulaire sèche, sont par exemple les excipients qu'elle comprend, qui sont aptes à contrarier, voire à rendre impossible (ou empêcher) le broyage des microparticules contenant le PA.

La forme médicamenteuse selon l'invention résout le problème posé et satisfait aux objectifs fixés, de façon efficace, simple et économique, à l'aide de moyens physico-chimiques. Ces derniers sont totalement inoffensifs pour l'utilisateur normal. Ce sont des composés pharmacologiquement neutres (inertes), approuvés par la pharmacopée et par les autorités de santé publique chargées de délivrer les autorisations de mise sur le marché des médicaments.

### Description détaillée de l'invention

La présente invention concerne avantageusement des formes pharmaceutiques microparticulaires sèches dont la composition permet d'empêcher, ou tout au moins de rendre très difficile, le broyage des microparticules contenant le PA.

Dans un premier mode de réalisation de l'invention, la présente invention concerne une forme médicamenteuse orale et solide, caractérisée en ce qu'au moins une partie du principe actif (PA) qu'elle comprend est contenu dans des microparticules et en ce qu'elle comprend des moyens anti-broyage qui sont des excipients entrant dans la composition de la forme médicamenteuse et qui sont prévus pour contrarier, voire empêcher le broyage des microparticules de PA, de manière à éviter le mésusage, lesdits excipients comprenant au moins un agent mottant (M, A) choisi dans la classe des composés hydrophobes agissant comme agent liant à sec, de telle sorte que, sous cisaillement, l'agent mottant (M, A) soit apte à transformer la forme médicamenteuse solide en une pâte non pulvérulente, à l'exclusion des formes médicamenteuses thermoformées sans extrusion ou thermoformées sans changement de couleur par extrusion, comprenant une combinaison de:
- un ou plusieurs principe(s) actif(s),
- au moins un polymère naturel ou synthétique ayant une résistance à la rupture d'au moins 500 N, et
- au moins une cire ayant une résistance à la rupture d'au moins 500 N,
- et optionnellement des additifs pharmaceutiquement acceptables, ladite forme étant exempte de toute combinaison constituée par au moins l'agent mottant (M,A) et par au moins un agent viscosifiant (B)

La méthode de détermination de la résistance à la rupture est la suivante:

Pour vérifier si un polymère ou une cire peut être utilisé respectivement en tant que polymère naturel ou synthétique, ou cire ayant une résistance à la rupture d'au moins 500 N, le matériau est compressé pour former une tablette ayant un diamètre de 10 millimètres et une hauteur de 5 millimètres en utilisant une force de 150 N à une température qui correspond au moins au point de ramollissement du matériau et est étudié avec l'assistance d'un diagramme DSC (Calorimétrie Enthalpique Différentielle) du matériau. En utilisant des tablettes produites de cette manière, la résistance à la rupture est déterminée avec l'appareil décrit ci-après en accord avec la méthode de détermination de la résistance à la rupture des tablettes publiée dans la Pharmacopée Européenne 1997, page 143-144, méthode n°2.9.8.

L'appareil utilisé pour la mesure est un testeur de matériaux de la Société Zwick GmbH and Co modèle Zwick Z2.5. Ce testeur a un Fmax. de 2,5 kN avec une course de traverse maximum de 1150 mm fixée à un cadre par des colonnes et une vis de conduite. L'appareil est pourvu d'une zone libre de test arrière de 100 mm, une vitesse de test de 0,1 à 800 mm/min et un logiciel de contrôle de test. Il est muni d'un tampon de pression à engagements vissables avec un cylindre (diamètre 10 mm) et d'un capteur de force, F max. de 1 kN, diamètre 8 mm.

Les tablettes considérées comme résistantes à la rupture sous une charge spécifique incluent non seulement celles qui ne se sont pas cassées mais également celles qui peuvent avoir subi une déformation plastique sous l'action de la force exercée.

L'agent mottant A) est choisi dans la classe des composés hydrophobes agissant comme agent liant à sec, de préférence:
→ dans le groupe comprenant les huiles de coton, les huiles de soja, les huiles de palme, les huiles de ricin et les mélanges de tout ou partie de ces huiles; et/ou
→ dans le groupe des cires, et plus préférentiellement encore dans le sous-groupe des cires comprenant les huiles de coton hydrogénées, les huiles de soja hydrogénées, les huiles de palme hydrogénées, les béhénates de glycérol, les huiles de ricin hydrogénées, les tristéarines, les tripalmitines, les trimyristines, les cires jaunes, les graisses dures, les matières grasses laitières anhydres, les lanolines, les palmitostéarates de glycérol, les stéarates de glycérol, les macrogolglycérides d'acide laurique, les alcools cétyliques, les diisostéarates de polyglycéryle, les monostéarates de diéthylène glycol, les monostéarates d'éthylèneglycol, les omégas 3 et les mélanges de tout ou partie de ces cires; et/ou
→ dans le groupe des bases grasses pour suppositoires comprenant la glycérine, les triglycérides, les huiles de théobroma, les beurres de cacao et les mélanges de tout ou partie de ces produits
et l'agent viscosifiant B) est choisi dans les groupes de polymères suivants :
→ les polyacides acryliques et leurs dérivés, et/ou
→ les polyalkylènes glycols (e.g. polyéthylène glycol), et/ou
→ les polyvinylpyrrolidones, et/ou
→ les gélatines, et/ou
→ les polysaccharides, de préférence dans le sous-groupe comprenant: l'alginate de sodium, les pectines, les guars, les xanthanes, les carraghénanes, les gellanes et les dérivés de la cellulose (e.g. hydroxypropylméthylcellulose, méthylcellulose, hydroxyéthylcellulose, carboxyméthylcellulose),
et leurs mélanges.

De préférence, l'agent mottant (M) est choisi de telle sorte que, sous cisaillement, il soit apte à transformer la forme médicamenteuse solide en une pâte non pulvérulente rendant difficile le broyage des microparticules de PA.

Or, il est bien connu que l'extraction d'un composé à concentrer d'une pâte visqueuse est extrêmement difficile.

L'agent mottant (M) est choisi dans la classe des composés hydrophobes agissant comme agent liant à sec, de préférence:
→ dans le groupe comprenant les huiles végétales :les huiles de coton, les huiles de soja, les huiles de palme, les huiles de ricin et les mélanges de tout ou partie de ces huiles; et/ou les huiles minérales et/ou
→ dans le groupe des cires, et plus préférentiellement encore dans le sous-groupe des cires comprenant les cires d'huiles de coton hydrogénées, les huiles de soja hydrogénées, les huiles de palme hydrogénées, les béhénates de glycérol, les huiles de ricin hydrogénées, les tristéarines, les tripalmitines, les trimyristines, les cires jaunes, les graisses dures, les matières grasses laitières anhydres, les lanolines, les palmitostéarates de glycérol, les stéarates de glycérol, les macrogolglycérides d'acide - laurique, les alcools- cétyliques, les diisostéarates de polyglycéryle, les monostéarates de diéthylène glycol, les monostéarates d'éthylèneglycol, les omégas 3 et les mélanges de tout ou partie de ces cires; et/ou
→ dans le groupe des bases grasses pour suppositoires comprenant la glycérine, les triglycérides, les huiles de théobroma, les beurres de cacao et les mélanges de tout ou partie de ces huiles.

Dans un deuxième mode de réalisation de l'invention, la forme médicamenteuse microparticulaire est caractérisée en ce que ses moyens anti-broyage comprennent des microbilles insolubles inertes, de diamètre moyen supérieur ou égal à 1,25 fois, de préférence 1,5 fois, et plus préférentiellement encore 2 fois, le diamètre moyen des microparticules de PA.

Ces microbilles insolubles en milieu aqueux ou hydroalcoolique, sont incompressibles. Du fait de leur taille plus importante que celle des microparticules renfermant le PA, les billes neutres supportent principalement les contraintes du broyage, protégeant ainsi les microparticules renfermant le PA . Elles rendent donc inefficaces les tentatives de broyage mécanique.

De préférence, les microbilles insolubles neutres sont choisies dans le groupe de matériaux suivants: les celluloses et leurs dérivés insolubles, les résines polyméthacryliques et leurs dérivés, les silices, le talc, la semoule de blé, la bentonite et leurs mélanges.

Dans un troisième mode de réalisation de l'invention, la forme médicamenteuse microparticulaire est caractérisée en ce ses moyens anti-broyage comprennent au moins un agent lubrifiant (L).

Le rôle de (des) l'agent(s) lubrifiant(s) est de limiter fortement, voir des supprimer l'abrasion des microparticules contenant le PA lors de leur broyage mécanique. L'agent lubrifiant ou glissant rend difficile le broyage de la forme médicamenteuse multiparticulaire en facilitant son écoulement, réduisant ainsi la contrainte de cisaillement appliquée au produit.

L'intérêt de l'agent lubrifiant ou glissant est de générer du glissement aux parois, le produit n'adhère donc pas à la paroi du broyeur ce qui empêche la transmission de la contrainte de cisaillement au principe actif présent dans les microparticules.

En présence de lubrifiant ou glissant, la poudre composée de microparticules de principe actif n'est pas cohésive et a un très bon écoulement.

L'agent lubrifiant est soit simplement mélangé à la forme pharmaceutique microparticulaire solide, soit encore directement déposé à la surface des microparticules contenant le PA par tout moyen connu de l'homme de l'art, par exemple par "spray coating".

A titre d'exemples non limitatifs d'agents lubrifiants ou glissants, on peut citer :
- Le stéarate de calcium ;
- Le palmitostéarate de glycérol ;
- Les huile végétales hydrogénées ;
- L'oxyde de magnésium;
- Les poloxamer ;
- Les polyethylène glycol :
- L'alcool polyvinylique ;
- Le benzoate de sodiums
- Les tensioatifs anioniques, cationiques ou non-ioniques;
- L'acide stérique ;
- L'amidon de maïs ;
- Le talc ;
- La silice colloïdale ;
- Le stearate de zinc ou de magnésium,
- et leurs mélanges.

La fraction massique d'agent lubrifiant dans la forme pharmaceutique solide selon l'invention est, par exemple, comprise entre 1 et 40 %.

Les microparticules selon l'invention et contenant le PA ont un diamètre -moyen inférieur ou égal à 1000 microns, de préférence compris entre 50 et 800 microns et de préférence encore compris entre 100 et 600 microns.

Les microparticules selon l'invention et contenant le PA peuvent être des microcapsules à libération modifiée de PA, c'est à dire des microparticules enrobée par un film polymère déposé selon les techniques connues de l'homme de l'art. On consultera sur cette question par exemple l'article « formes pharmaceutiques nouvelles » de Buri, Puisieux, Doelker et Benoit, Lavoisier 1985, p175-227 .

Avantageusement, la forme médicamenteuse selon l'invention n'est pas transformable en une forme sèche administrable par aspiration nasale.

Il est également à noter que cette forme médicamenteuse selon l'invention peut ne pas être transformable en une forme injectable.

Suivant une variante, la forme médicamenteuse selon l'invention comprend du PA à libération immédiate et/ou du PA à libération modifiée.

De préférence, au moins une partie des microparticules de PA de la forme médicamenteuse selon l'invention sont des microparticules, de préférence des microcapsules à libération modifiée de PA.

Avantageusement, les microparticules de PA ou les microcapsules de PA ont un diamètre moyen inférieur ou égal à 1000 microns de préférence compris entre 50 et 800 microns et de préférence encore compris entre 100 et 600 microns.

Suivant une autre caractéristique remarquable de la forme médicamenteuse selon l'invention, l'extraction du PA par mastication et/ou broyage n'est pas efficace.

Le PA mis en oeuvre appartient par exemple à au moins l'une des familles de substances actives suivantes : amphétamines, analgésiques, anorexigènes, antalgiques, antidépresseurs, antiépileptiques, antimigraineux, antiparkinsoniens, antitussifs, anxiolytiques, barbituriques, benzodiazépines, hypnotiques, laxatifs, neuroleptiques, opiacés, psychostimulants, psychotropes, sédatifs, stimulants.

Plus précisément encore, le PA mis en oeuvre est choisi parmi les composés suivants:

Méthylphénidate, Fentanyl, Alfentanyl, Pentazocine, Péthidine, Phénopéridine, Rémifentanil, Sufentanil, Acétorphine, Acétylalphaméthylfentanyl, Acétylméthadol, Alfentanil, Allylprodine, Alphacétylméthadol, Alphaméprodine, Alphaméthadol, Alphaméthylfentanyl, Alpha-méthylthofentanyl, Alphaprodine, Aniléridine, atropine, Benzéthidine, Benzylmorphine, Béta-hydroxyfentanyl, Béta-hydroxy-méthyl-3-fentanyl Bétacétylméthadol, Bétaméprodine, Bétaméthadol, Bétaprodine, Bezitramide, buprénorphine, Butyrate de dioxaphétyl, Cannabis, Cétobémidone, Clonitazène, codéine, Coca, Cocaïne, Codoxime, Concentré de paille de pavot, Désomorphine, Dextromoramide, Diampromide, Diéthylthiambutène, Difénoxine, Dihydroétorphine, Dihydromorphine, Diménoxadol, Dimépheptanol, Diméthylthiambutène, Diphénoxylate, Dipipanone, Drotébanol, Ecgonine, éphédrine, Ethylméthylthiambutène, Etonitazène, Etorphine, Etoxéridine, Furéthidine, Héroine, Hydrocidone, Hydromorphinol, Hydromorphone, Hydroxypéthidine, Isométhadone, Lévométhorphane, Lévomoramide, Lévophénacylmorphane, Lévorphanol, meperidine, Métazocine, Méthadone, Méthyldésorphine, Méthyldihydromorphine, Méthyl-3-thiofentanyl, Méthyl-3-fentanyl, Métopon, Moramide, Morphéridine, morphine, MPPP, Myrophine, Nicomorphine, Noracyméthadol, Norlévorphanol, Norméthadone, Normorphine, Norpipanone, Opium, Oxycodone, Oxymorphone, Para-fluorofentanyl, PEPAP, Phénampromide, Phénazocine, Phénomorphane, Piminodine, Piritramide, Proheptazine, propanolol, Propéridine, Racéméthorphane, Racémoramide, Racémorphane, Thébacone, Thébaïne, Thiofentanyl, Tilidine, Trimépéridine, Acétyldihydrocodéine, Dextropropoxyphène, Dihydrocodéine, Ethylmorphine, Nicocodine, Nicodicodine, Norcodéine, Pholcodine, Propiram, et leurs mélanges.

Les modes de réalisation ci-dessus peuvent être pris isolément ou en q combinaison les uns avec les autres.

### Exemple 1 :

Préparation de microparticules d'acyclovir (principe actif modèle) :
Etape 1 : Granulé
   15 g d'acyclovir, 25 g de PEG 40-huile de ricin hydrogénée et 30 g de Povidone sont préalablement solubilisés dans un mélange eau / acétone / isopropanol (5/57/38 m/m). Cette solution est ensuite pulvérisée sur 800 g de sphères de cellulose (de diamètre compris entre 100 et 200 µm) dans un appareil à lit d'air fluidisé Glatt GPC-G1.
Etape 2 : Enrobage
   50 g de granulés obtenus précédemment sont enrobés par 6,5 g d'éthylcellulose, 0,5 g d'huile de ricin, 0,5 g de PEG40-huile de ricin hydrogénée (BASF) et 2,5 g de povidone dissous dans un mélange acétone / isopropanol (60/40 m/m), dans un appareil à lit d'air fluidisé miniGlatt.

Le diamètre moyen des microparticules obtenues est de 180 µm. Ces microparticules sont quasiment sphériques.

### Exemple 2 :

35 g de PEG 6000 et 5 g de stéarate de magnésium sont dispersés dans 160 g d'isopropanol. Cette dispersion est ensuite pulvérisée sur 40 g de microparticules obtenues à l'issue de la deuxième étape de l'exemple 1.

Lors du cisaillement de ces objets, la couche contenant le PEG 6000 et le Stéarate de magnésium protège la particule de principe actif en réduisant les effets du cisaillement.

## Revendications

1. Forme médicamenteuse orale et solide, **caractérisée en ce qu'**au moins une partie du principe actif (PA) qu'elle comprend est contenu dans des microparticules et **en ce qu'**elle comprend des moyens anti-broyage qui sont des excipients entrant dans la composition de la forme médicamenteuse et qui sont prévus pour contrarier, voire empêcher le broyage des microparticules de PA, de manière à éviter le mésusage, lesdits excipients comprenant au moins un agent mottant (M, A) qui est un composé hydrophobe agissant comme agent liant à sec, choisi :
- dans le groupe constitué par les huiles de coton, les huiles de soja, les huiles de palme, les huiles de ricin et leurs mélanges, et/ou
- dans le groupe des cires constitué par les huiles de coton hydrogénées, les huiles de soja hydrogénées, les huiles de palme hydrogénées, les béhénates de glycérol, les huiles de ricin hydrogénées, les tristéarines, les tripalmitines, les trimyristines, les cires jaunes, les graisses dures, les matières grasses laitières anhydres, les lanolines, les palmitostéarates de glycérol, les stéarates de glycérol, les macrogolglycérides, d'acide laurique, les alcools cétyliques, les diisostéarates de polyglycéryle, les monostéarates de diéthylène glycol, les monostéarates d'éthylèneglycol, les omégas 3 et leurs mélanges, et/ou
- dans le groupe des bases grasses pour suppositoires constitué par la glycérine, les triglycérides, les huiles de théobroma, les beurres de cacao ;
- et leurs mélanges ;
de telle sorte que, sous cisaillement, l'agent mottant (M, A) soit apte à transformer la forme médicamenteuse solide en une pâte non pulvérulente,
à l'exclusion des formes médicamenteuses thermoformées sans extrusion ou thermoformées sans changement de couleur par extrusion, comprenant une combinaison de:
- un ou plusieurs principe(s) actif(s),
- au moins un polymère naturel ou synthétique ayant une résistance à la rupture d'au moins 500 N,
- au moins une cire ayant une résistance à la rupture d'au moins 500 N,
- et optionnellement des additifs pharmaceutiquement acceptables. **caractérisée en ce qu'**elle est exempte de toute combinaison constituée par au moins l'agent mottant (M, A) et par au moins un agent viscosifiant (B).

2. Forme médicamenteuse selon la revendication 1, dans laquelle ledit au moins un agent viscosifiant (B) est choisi dans les groupes de polymères suivants :
- les polyacides acryliques et leurs dérivés, et/ou
- les polyalkylènes glycols (e.g. polyéthylène glycol), et/ou
- les polyvinylpyrrolidones, et/ou
- les gélatines, et/ou
- les polysaccharides, de préférence dans le sous-groupe comprenant: l'alginate de sodium, les pectines, les guars, les xanthanes, les carraghénanes, les gellanes et les dérivés de la cellulose (e.g. hydroxypropylméthylcellulose, méthylcellulose, hydroxyéthylcellulose, carboxyméthylcellulose),
- et leurs mélanges.

3. Forme médicamenteuse selon l'une quelconque des revendications précédentes, **caractérisée en ce qu'**elle est constituée par une forme sèche.

4. Forme médicamenteuse selon l'une des revendications précédentes, **caractérisée en ce que** les moyens anti-broyage comprennent des microbilles insolubles inertes, de diamètre moyen supérieur ou égal à 1,25 fois, de préférence 1,5 fois, et plus préférentiellement encore 2 fois, le diamètre moyen des microparticules de PA.

5. Forme médicamenteuse selon la revendication 4, **caractérisée en ce que** les microbilles insolubles neutres sont choisies dans le groupe de matériaux suivants: les celluloses et leurs dérivés insolubles, les résines polyméthacryliques et leurs dérivés, les silices, le talc, la semoule de blé, la bentonite et leurs mélanges.

6. Forme médicamenteuse selon l'une quelconque des revendications précédentes, **caractérisée en ce que** les moyens anti-broyage comprennent au moins un agent lubrifiant (L).

7. Forme médicamenteuse selon la revendication 6, **caractérisée en ce que** l'agent lubrifiant (L) est choisi dans le groupe comprenant:
- Le stéarate de calcium ;
- Le palmitostéarate de glycérol ;
- Les huile végétales hydrogénées ;
- L'oxyde de magnésium ;
- Les poloxamer ;
- Les polyethylène glycol ;
- L'alcool polyvinylique ,
- Le benzoate de sodium ;
- Les tensioatifs anioniques, cationiques ou non-ioniques ;
- L'acide stéarique ;
- L'amidon de maïs ;
- Le talc ;
- La silice colloïdale ;
- Le stéarate de zinc ou de magnésium,
- et leurs mélanges.

8. Forme médicamenteuse selon l'une quelconque des revendications précédentes, **caractérisée en ce qu'**elle n'est pas transformable en une forme injectable ou une forme sèche administrable par aspiration nasale.

9. Forme médicamenteuse selon l'une quelconque des revendications précédentes, **caractérisée en ce qu'**elle comprend du PA à libération immédiate et/ou du PA à libération modifiée.

10. Forme médicamenteuse selon l'une quelconque des revendications précédentes, **caractérisée en ce qu'**au moins une partie des microparticules de PA sont des microparticules, de préférence des microcapsules, à libération modifiée de PA.

11. Forme médicamenteuse selon l'une quelconque des revendications précédentes, **caractérisée en ce que** les microparticules de PA ou les microcapsules de PA ont un diamètre moyen inférieur ou égal à 1 000 microns, de préférence compris entre 50 et 800 microns et de préférence encore compris entre 100 et 600 microns.

12. Forme médicamenteuse selon l'une des revendications précédentes, **caractérisée en ce que** le PA mis en oeuvre appartient à au moins l'une des familles de substances actives suivantes: amphétamines, analgésiques, anorexigènes, antalgiques, antidépresseurs, antiépileptiques, antimigraineux, antiparkinsoniens, antitussifs, anxiolytiques, barbituriques, benzodiazépines, hypnotiques, laxatifs, neuroleptiques, opiacés, psychostimulants, psychotropes, sédatifs, stimulants.

13. Forme médicamenteuse selon l'une des revendications précédentes, **caractérisée en ce que** le PA mis en oeuvre est choisi parmi les composés suivants: Méthylphénidate, Fentanyl, Alfentanyl, Pentazocine, Péthidine, Phénopéridine, Rémifentanil, Sufentanil, Acétorphine, Acétylalphaméthylfentanyl, Acétylméthadol, Alfentanil, Allylprodine, Alphacétylméthadol, Alphaméprodine, Alphaméthadol, Alphaméthylfentanyl, Alpha-méthylthofentanyl, Alphaprodine, Aniléridine, Atropine, Benzéthidine Benzylmorphine, Béta-hydroxyfentanyl, Béta-hydroxy-méthyl-3-fentanyl, Bétacétylméthadol, Bétaméprodine, Bétaméthadol, Bétaprodine, Bezitramide, Buprénorphine, Butyrate de dioxaphétyl, Cannabis, Cétobémidone, Clonitazène, Codéine, Coca, Cocaïne, Codoxime, Concentré de paille de pavot, Désomorphine, Dextromoramide, Diampromide, Diéthylthiambutène, Difénoxine, Dihydroétorphine, Dihydromorphine, Diménoxadol, Dimépheptanol, Diméthylthiambutène, Diphénoxylate, Dipipanone, Drotébanol, Ecgonine, Ephédrine, Ethylméthylthiambutène, Etonitazène, Etorphine Etoxéridine, Furéthidine, Héroïne, Hydrocodone, Hydromorphinol, Hydromorphone, Hydroxypéthidine, Isométhadone, Lévométhorphane, Lévomoramide, Lévophénacylmorphane, Lévorphanol, Meperidine, Métazocine, Méthadone, Méthyldésorphine, Méthyldihydromorphine, Méthyl-3-thiofentanyl, Méthyl-3-fentanyl, Métopon, Moramide, Morphéridine, Morphine, MPPP, Myrophine, Nicomorphine, Noracyméthadol, Norlévorphanol, Norméthadone, Normorphine, Norpipanone, Opium, Oxycodone, Oxymorphone, Para-fluorofentanyl, PEPAP, Phénampromide, Phénazocine, Phénomorphane, Piminodine, Piritramide, Proheptazine, Propanolol, Propéridine, Racéméthorphane, Racémoramide, Racémorphane, Thébacone, Thébaïne, Thiofentanyl, Tilidine, Trimépéridine, Acétyldihydrocodéine, Dextropropoxyphène, Dihydrocodéine, Ethylmorphine, Nicocodine, Nicodicodine, Norcodéine, Pholcodine, Propiram, et leurs mélanges.

## Claims

1. An oral and solid drug form, **characterized in that** at least part of the active ingredient (AI) that it comprises is contained in microparticles, and **in that** it comprises anticrushing means which are excipients involved in the composition of the drug form and are intended to impede, or even prevent the crushing of the microparticles of AI, so as to prevent misuse, said excipients comprising at least one caking agent (M, A) which is a hydrophobic compounds which act as a dry binder, chosen:
→ from the group consisting of cottonseed oils, soybean oils, palm oils, castor oils and mixtures of all or some of these oils, and/or
→ from the group of waxes consisting of hydrogenated cottonseed oils, hydrogenated soybean oils, hydrogenated palm oils, glyceryl behenates, hydrogenated castor oils, tristearins, tripalmitins, trimyristins, yellow waxes, hard fats, anhydrous dairy fats, lanolins, glyceryl palmitostearates, glyceryl stearates, lauric acid macrogolglycerides, cetyl alcohols, polyglyceryl diisostearates, diethylene glycol monostearates, ethylene glycol monostearates, omegas 3 and mixtures thereof, and/or
→ from the group of fatty bases for suppositories consisting of glycerol, triglycerides, theobroma oils, cocoa butters and mixtures thereof;
so as, under shearing, the caking agent (M, A) is capable of converting the solid drug form into a nonpulverulent paste, excluding the dosage forms thermoformed without extrusion or thermoformed without coloration changing by extrusion, comprising a combination of:
- one or more active ingredient(s),
- at least one natural or synthetic polymer having a breaking strength of at least 500 N,
- at least one wax having a breaking strength of at least 500 N,
- and optionally pharmaceutically acceptable excipients,
**characterized in that** it is free of any combination consisting of at least one caking agent (M, A) and of at least one viscosifying agent (B).

2. The drug form as claimed in claim 1, **characterized in that** the at least one viscosifying agent (B) is chosen from the following groups of polymers:
→ polyacrylic acids and derivatives thereof, and/or
→ polyalkylene glycols (e.g. polyethylene glycol), and/or
→ polyvinylpyrrolidones, and/or
→ gelatins, and/or
→ polysaccharides, preferably from the subgroup comprising: sodium alginate, pectins, guars, xanthans, carrageenans, gellans and cellulose derivatives (e.g. hydroxypropylmethylcellulose, methylcellulose, hydroxyethylcellulose, carboxymethylcellulose),
and mixtures thereof.

3. Drug form according to any of the previous claims, **characterized in that** it consists of a dry form.

4. Drug form according to any of the previous claims, **characterized in that** the anticrushing means comprise insoluble inert microbeads which have an average diameter of greater than or equal to 1.25 times, preferably 1.5 times, and even more preferably twice, the average diameter of the microparticles of AI.

5. Drug form according to claim 4, **characterized in that** the insoluble neutral microbeads are chosen from the group of following substances: celluloses and insoluble derivatives thereof, polymethacrylic resins and derivatives thereof, silicas, talc, semolina, bentonite and mixtures thereof.

6. Drug form according to any of the previous claims, **characterized in that** the anti-crushing means comprise at least one lubricant (L).

7. Drug form according to claim 6, **characterized in that** the lubricant (L) is chosen from the group comprising:
- calcium stearate;
- glyceryl palmitostearate;
- hydrogenated plant oils;
- magnesium oxide;
- poloxamers;
- polyethylene glycols;
- polyvinyl alcohol;
- sodium benzoate;
- anionic, cationic or nonionic surfactants;
- stearic acid;
- corn starch;
- talc;
- colloidal silica;
- zinc stearate, magnesium stearate,
- and mixtures thereof.

8. Drug form according to any of the previous claims, **characterized in that** it cannot be converted into an injectable form or a dry form that can be administered by nasal aspiration.

9. Drug form according to any of the previous claims, **characterized in that** it comprises immediate-release AI and/or modified-release AI.

10. The drug form as claimed in any one of the preceding claims, **characterized in that** at least some of the microparticles of AI are microparticles, preferably microcapsules, for modified release of AI.

11. Drug form according to any of the previous claims, **characterized in that** the microparticles of AI or the microcapsules of AI have an average diameter of less than or equal to 1000 microns, preferably between 50 and 800 microns, and even more preferably between 100 and 600 microns.

12. Drug form according to any of the previous claims, **characterized in that** the AI used belongs to at least one of the following families of active substances: amphetamines, analgesics, appetite suppressants, antidepressants, antiepileptics, antimigraine agents, antiparkinsonian agents, antitussives, anxiolytics, barbiturates, benzodiazepines, hypnotics, laxatives, neuroleptics, opiates, psychostimulants, psychotropic agents, sedatives and stimulants.

13. Drug form according to any of the previous claims, **characterized in that** the AI used is chosen from the following compounds: methylphenidate, Fentanyl, Alfentanyl, Pentazocine, Pethidine, Phenoperidine, Remifentanil, Sufentanil, Acetorphine, Acetylalphamethylfentanyl, Acetylmethadol, Alfentanil, Allylprodine, Alphacetylmethadol, Alphameprodine, Alphamethadol, Alphamethylfentanyl, Alpha-methylthiofentanyl, Alphaprodine, Anileridine, atropine, Benzethidine, Benzylmorphine, Beta-hydroxyfentanyl, Beta-hydroxymethyl-3-fentanyl, Betacetylmethadol, Betameprodine, Betamethadol, Betaprodine, Bezitramide, buprenorphine, Dioxaphetyl butyrate, Cannabis, Ketobemidone, Clonitazene, codeine, Coca, Cocaine, Codoxime, Concentrate of poppy straw, Desomorphine, Dextromoramide, Diampromide, Diethylthiambutene, Difenoxine, Dihydroetorphine, Dihydromorphine, Dimenoxadol, Dimepheptanol, Dimethylthiambutene, Diphenoxylate, Dipipanone, Drotebanol, Ecgonine, ephedrine, Ethylmethylthiambutene, Etonitazene, Etorphine, Etoxeridine, Furethidine, Heroin, Hydrocodone, Hydromorphinol, Hydromorphone, Hydroxypethidine, Isomethadone, Levomethorphane, Levomoramide, Levophenacylmorphane, Levorphanol, meperidine, Metazocine, Methadone, Methyldesorphine, Methyldihydromorphine, Methyl-3-thiofentanyl, Methyl-3-fentanyl, Metopon, Moramide, Morpheridine, morphine, MPPP, Myrophine, Nicomorphine, Noracymethadol, Norlevorphanol, Normethadone, Normorphine, Norpipanone, Opium, Oxycodone, Oxymorphone, Para-fluorofentanyl, PEPAP, Phenampromide, Phenazocine, Phenomorphane, Piminodine, Piritramide, Proheptazine, propanolol, Properidine, Racemethorphane, Racemoramide, Racemorphane, Thebacone, Thebaine, Thiofentanyl, Tilidine, Trimeperidine, Acetyldihydrocodeine, Dextropropoxyphene, Dihydrocodeine, Ethylmorphine, Nicocodine, Nicodicodine, Norcodeine, Pholcodine, Propiram, and mixtures thereof.

## Patentansprüche

1. Orale und feststoffliche Arzneiform, **dadurch gekennzeichnet, dass** mindestens ein Teil des Wirkstoffs (WS), welchen sie umfasst, in Mikropartikeln enthalten ist, und dass sie Anti-Zerkleinerungsmittel umfasst, wobei es sich um pharmazeutische Hilfsstoffe handelt, die Teil der Zusammensetzung der Arzneiform sind und die dafür vorgesehen sind, die Zerkleinerung der WS-Mikropartikel zu erschweren oder sogar zu verhindern, sodass ein Missbrauch vermieden wird, wobei die pharmazeutischen Hilfsstoffe mindestens ein Zusammenbackmittel (M, A) umfassen, wobei es sich um eine hydrophobe Verbindung handelt, die als Trockenbindemittel wirkt und aus Folgendem ausgewählt ist:
- aus der Gruppe, die aus den Baumwollölen, den Sojaölen, den Palmölen, den Rizinusölen und deren Mischungen besteht, und/oder
- aus der Gruppe der Wachse, die aus den hydrierten Baumwollölen, den hydrierten Sojaölen, den hydrierten Palmölen, den Glycerinbehensäureestern, den hydrierten Rizinusölen, den Tristearinen, den Tripalmitinen, den Trimyristinen, den gelben Wachsen, den Hartfetten, den wasserfreien Milchfetten, den Lanolinen, den Glycerinpalmitostearaten, den Glycerinstearaten, den Laurinsäuremacrogolglyceriden, den Cetylalkoholen, den Polyglyceryldiisostearaten, den Diethylenglykolmonostearaten, den Ethylenglykolmonostearaten, den Omega-3-Stoffen und deren Mischungen besteht, und/oder
- aus der Gruppe der Fettbasis für Suppositorien, die aus Glycerin, den Triglyceriden, den Theobromaölen, den Kakaobuttern besteht;
- und aus deren Mischungen;
derart, dass das Zusammenbackmittel (M, A) dazu befähigt ist, die feststoffliche Arzneiform unter Scherwirkung in eine nicht-pulverförmige Paste umzuwandeln,
mit Ausnahme von Arzneiformen, die ohne Extrusion thermogeformt werden oder ohne Verfärbung durch Extrusion thermogeformt werden, wobei sie eine Kombination aus Folgendem umfassen:
- einem oder mehreren Wirkstoff(en),
- mindestens einem natürlichen oder synthetischen Polymer mit einer Bruchfestigkeit von mindestens 500 N,
- mindestens einem Wachs mit einer Bruchfestigkeit von mindestens 500 N,
- und möglicherweise pharmazeutisch unbedenklichen Zusatzstoffen,
und die **dadurch gekennzeichnet ist, dass** sie frei von jedweder Kombination ist, die aus mindestens dem Zusammenbackmittel (M, A) und aus mindestens einem viskositätserhöhenden Mittel (B) besteht.

2. Arzneiform nach Anspruch 1, wobei das mindestens eine viskositätserhöhende Mittel (B) aus der Gruppe der folgenden Polymere ausgewählt ist:
- den Polyacrylsäuren und deren Derivaten, und/oder
- den Polyalkylenglykolen (z.B. Polyethylenglykol), und/oder
- den Polyvinylpyrrolidonen, und/oder
- den Gelatinen, und/oder
- den Polysacchariden, vorzugsweise aus der Untergruppe, die Folgendes umfasst: Natriumalginat, Pektine, Guarstoffe, Xanthane, Carrageene, Gellanstoffe und Cellulosederivate (z.B. Hydroxypropylmethylcellulose, Methylcellulose, Hydroxyethylcellulose, Carboxymethylcellulose),
- und deren Mischungen.

3. Arzneiform nach einem beliebigen der vorhergehenden Ansprüche, **dadurch gekennzeichnet, dass** sie aus einer trockenen Form besteht.

4. Arzneiform nach einem der vorhergehenden Ansprüche, **dadurch gekennzeichnet, dass** die Anti-Zerkleinerungsmittel inerte unlösliche Mikrokügelchen umfassen, mit einem mittleren Durchmesser, der mindestens 1,25-mal, vorzugsweise 1,5-mal und mit noch größerem Vorzug 2-mal so groß wie der mittlere Durchmesser der WS-Mikropartikel ist.

5. Arzneiform nach Anspruch 4, **dadurch gekennzeichnet, dass** die neutralen unlöslichen Mikrokügelchen aus der Gruppe der folgenden Materialien ausgewählt sind: den Cellulosen und ihren unlöslichen Derivaten, den Polymethacrylharzen und ihren Derivaten, den Kieselsäuren, Talkum, Weizengrieß, Bentonit und deren Mischungen.

6. Arzneiform nach einem beliebigen der vorhergehenden Ansprüche, **dadurch gekennzeichnet, dass** die Anti-Zerkleinerungsmittel mindestens ein Schmiermittel (L) umfassen.

7. Arzneiform nach Anspruch 6, **dadurch gekennzeichnet, dass** das Schmiermittel (L) aus der Gruppe gewählt ist, die Folgendes umfasst:
- Calciumstearat;
- Glycerinpalmitostearat;
- hydrierte pflanzliche Öle;
- Magnesiumoxid;
- Poloxamer-Stoffe;
- Polyethylenglykol-Stoffe;
- Polyvinylalkohol;
- Natriumbenzoat;
- anionische, kationische oder nichtionische Tenside;
- Stearinsäure;
- Maisstärke;
- Talkum;
- kolloidale Kieselsäure;
- zink- oder Magnesiumstearat,
- und deren Mischungen.

8. Arzneiform nach einem beliebigen der vorhergehenden Ansprüche, **dadurch gekennzeichnet, dass** sie nicht in eine injizierbare Form oder eine trockene Form, die durch Ansaugen in die Nase verabreichbar ist, umgewandelt werden kann.

9. Arzneiform nach einem beliebigen der vorhergehenden Ansprüche, **dadurch gekennzeichnet, dass** sie WS zur sofortigen Freisetzung und/oder WS zur modifizierten Freisetzung umfasst.

10. Arzneiform nach einem beliebigen der vorhergehenden Ansprüche, **dadurch gekennzeichnet, dass** es sich bei mindestens einem Teil der WS-Mikropartikel um Mikropartikel, vorzugsweise Mikrokapseln, zur modifizierten WS-Freisetzung handelt.

11. Arzneiform nach einem beliebigen der vorhergehenden Ansprüche, **dadurch gekennzeichnet, dass** die WS-Mikropartikel oder die WS-Mikrokapseln einen mittleren Durchmesser haben, der kleiner oder gleich 1000 Mikrometern ist, vorzugsweise im Bereich von 50 bis 800 Mikrometer und mit noch größerem Vorzug im Bereich von 100 bis 600 Mikrometern liegt.

12. Arzneiform nach einem der vorhergehenden Ansprüche, **dadurch gekennzeichnet, dass** der eingesetzte WS mindestens einer der folgenden Wirkstoff-Familien angehört: Amphetamine, schmerzstillende Mittel, Appetitzügler, schmerzlindernde Mittel, Antidepressiva, Antiepileptika, Migränemittel, Mittel gegen die Parkinson-Krankheit, Hustenstiller, Angstlöser, Barbiturate, Benzodiazepine, Schlafmittel, Abführmittel, Neuroleptika, Opiate, Psychostimulantien, psychotrope Stoffe, Beruhigungsmittel, Stimulantien.

13. Arzneiform nach einem der vorhergehenden Ansprüche, **dadurch gekennzeichnet, dass** der eingesetzte WS aus den folgenden Verbindungen ausgewählt ist: Methylphenidat, Fentanyl, Alfentanyl, Pentazocin, Pethidin, Phenoperidin, Remifentanil, Sufentanil, Acetorphin, Acetylalphamethylfentanyl, Acetylmethadol, Alfentanil, Allylprodin, Alphacetylmethadol, Alphameprodin, Alphamethadol, Alphamethylfentanyl, Alpha-methylthofentanyl, Alphaprodin, Anileridin, Atropin, Benzethidin Benzylmorphin, Betahydroxyfentanyl, Beta-hydroxymethyl-3-fentanyl, Betacetylmethadol, Betameprodin, Betamethadol, Betaprodin, Bezitramid, Buprenorphin, Dioxaphetylbutyrat, Cannabis, Cetobemidon, Clonitazen, Codein, Coca, Cocain, Codoxim, Schlafmohnstängel-Konzentrat, Desomorphin, Dextromoramid, Diampromid, Diethylthiambuten, Difenoxin, Dihydroetorphin, Dihydromorphin, Dimenoxadol, Dimepheptanol, Dimethylthiambuten, Diphenoxylat, Dipipanon, Drotebanol, Ecgonin, Ephedrin, Ethylmethylthiambuten, Etonitazen, Etorphin Etoxeridin, Furethidin, Heroin, Hydrocodon, Hydromorphinol, Hydromorphon, Hydroxypethidin, Isomethadon, Levomethorphan, Levomoramid, Levophenacylmorphan, Levorphanol, Meperidin, Metazocin, Methadon, Methyldesorphin, Methyldihydromorphin, Methyl-3-thiofentanyl, Methyl-3-fentanyl, Metopon, Moramid, Morpheridin, Morphin, MPPP, Myrophin, Nicomorphin, Noracymethadol, Norlevorphanol, Normethadon, Normorphin, Norpipanon, Opium, Oxycodon, Oxymorphon, Para-fluorfentanyl, PEPAP, Phenampromid, Phenazocin, Phenomorphan, Piminodin, Piritramid, Proheptazin, Propanolol, Properidin, Racemethorphan, Racemoramid, Racemorphan, Thebacon, Thebain, Thiofentanyl, Tilidin, Trimeperidin, Acetyldihydrocodein, Dextropropoxyphen, Dihydrocodein, Ethylmorphin, Nicocodin, Nicodicodin, Norcodein, Pholcodin, Propiram, und deren Mischungen.
